(19) Europäisches Patentamt
European Patent Office
Office européen des brevets

(11) EP 3 318 588 A1

(12) **EUROPEAN PATENT APPLICATION**
published in accordance with Art. 153(4) EPC

(43) Date of publication:
**09.05.2018 Bulletin 2018/19**

(21) Application number: **16817789.7**

(22) Date of filing: **22.06.2016**

(51) Int Cl.:
***C08G 18/32*** *(2006.01)*

(86) International application number:
**PCT/JP2016/068534**

(87) International publication number:
**WO 2017/002681 (05.01.2017 Gazette 2017/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **01.07.2015 JP 2015132556**

(71) Applicant: **TOYO TIRE & RUBBER CO., LTD.**
**Itami-shi**
**Hyogo 664-0847 (JP)**

(72) Inventor: **ISEKI,Seiji**
**Itami-shi,**
**Hyogo 6640847 (JP)**

(74) Representative: **Sajda, Wolf E.**
**Meissner Bolte Patentanwälte**
**Rechtsanwälte Partnerschaft mbB**
**Postfach 86 06 24**
**81633 München (DE)**

# (54) LIQUID-CRYSTAL URETHANE COMPOUND, THERMALLY RESPONSIVE LIQUID-CRYSTAL POLYURETHANE ELASTOMER, AND PRODUCTION METHOD THEREFOR

(57) The purpose of the present invention is to provide: a thermally responsive liquid-crystal polyurethane elastomer which is capable of being industrially continuously molded, and which is liquid crystal and has rubber elasticity at low temperatures (in the vicinity of room temperature); and a production method and a starting material therefor. This liquid-crystal urethane compound is obtained by reacting a mesogenic group-containing compound having at least active hydrogen groups, an alkylene oxide and/or a styrene oxide, and a diisocyanate compound.



Printed by Jouve, 75001 PARIS (FR)

**Description**

## TECHNICAL FIELD

The present invention relates to a liquid-crystal urethane compound, a thermally responsive liquid-crystal polyurethane elastomer, and a production method therefor.

## BACKGROUND ART

A liquid-crystal elastomer is a hybrid material of liquid-crystals and elastomers. The liquid-crystal elastomer shows a characteristic response behavior in such a manner that the liquid-crystal elastomer is extended in the orientation direction by applying an external stimulus such as heat, light, electric field, and magnetic field to increase the degree of liquid-crystal orientation, but the liquid-crystal elastomer is shrunk by removing such a stimulus to decrease the degree of liquid-crystal orientation. Thus, applications of such an elastomer to various fields such as actuators and the like have been attempted.

For example, Patent Document 1 discloses a high-molecular liquid-crystal polyurethane exhibiting liquid crystallinity, which is obtained by reacting a bis($\omega$-hydroxy-alkyleneoxy)biphenyl with 1,4-phenylene diisocyanate.

Further, Patent Document 2 discloses a polymer liquid-crystal polyurethane obtained by polymerizing a diol component having a mesogenic group with a trans-1, 4-cyclohexane diisocyanate.

However, in the conventional liquid-crystal polyurethane, the temperature at which a liquid-crystal is developed is very high and it was difficult to develop liquid crystallinity at a low temperature (near room temperature). Further, the conventional liquid-crystal polyurethane did not exhibit rubbery elasticity due to increased fluidity when a liquid-crystal is developed therein. Further, the conventional mesogenic diol as a starting material has a high temperature at which liquid crystallinity is exhibited and was difficult to produce a liquid-crystal polyurethane without a solvent.

In order to solve the above problem, the present inventor proposed a thermally responsive material having liquid-crystallinity and rubber elasticity at low temperatures (in the vicinity of room temperature) (unpublished at the time of filing).

However, there was a problem that the thermally responsive material was difficult to be industrially molded continuously.

## PRIOR ART DOCUMENTS

## PATENT DOCUMENTS

Patent Document 1: JP-A-H5-170860
Patent Document 2: JP-A-H7-258369

## SUMMARY OF THE INVENTION

## PROBLEMS TO BE SOLVED BY THE INVENTION

The purpose of the present invention is to provide a thermally responsive liquid-crystal polyurethane elastomer which is capable of being industrially molded continuously and which has liquid crystallinity and rubber elasticity at low temperatures (in the vicinity of room temperature), and a production method and a starting material therefor.

## MEANS FOR SOLVING THE PROBLEMS

The inventors of the present invention have made extensive and intensive studies to solve the above problems, and, as a result, have found that the purpose of the present invention can be achieved by the following thermally responsive liquid-crystal polyurethane elastomer, and production method and starting material therefor. The present invention has been completed based on these findings.

The present invention relates to a liquid-crystal urethane compound obtained by reacting a mesogenic group-containing compound having at least an active hydrogen group, an alkylene oxide and/or a styrene oxide, and a diisocyanate compound.

The inventor of the present invention found that addition of an alkylene oxide and/or a styrene oxide to a mesogenic group-containing compound having an active hydrogen group reduces the thermal stability of the mesogenic

group, so that the temperature range exhibiting the liquid-crystallinity of the liquid-crystal urethane compound is lowered. The liquid-crystal urethane compound of the present invention is a compound prepared by reacting these starting materials with a diisocyanate compound to prepolymerize them. By using the liquid-crystal urethane compound, it is possible to continuously form a thermally responsive liquid-crystal polyurethane elastomer while reaction-curing the urethane compound in the absence of a solvent in a state in which liquid-crystallinity is exhibited. By the reaction-curing in a state in which liquid-crystallinity is exhibited, it is possible to inhibit the crystallinity of a mesogen and prevent the formation of a crystalline phase.

It is preferable that the mesogenic group-containing compound is a compound represented by the following general formula (1) :

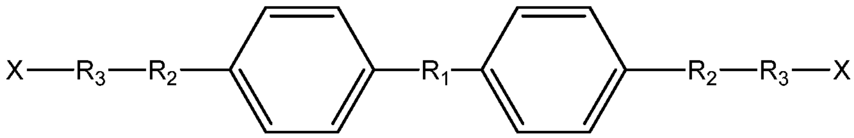

(1)

wherein X is an active hydrogen group, $R_1$ is a single bond, -N=N-, -CO-, -CO-O-, or -CH=N-, $R_2$ is a single bond or -O-, and $R_3$ is a single bond or an alkylene group having 1 to 20 carbon atoms, provided that the compound when $R_2$ is -O- and $R_3$ is a single bond is excluded.

In addition, it is preferable that the alkylene oxide is at least one member selected from the group consisting of ethylene oxide, propylene oxide, and butylene oxide.

In addition, it is preferable that 2 to 10 moles of the alkylene oxide and/or the styrene oxide add to 1 mole of the mesogenic group-containing compound. When the number of moles added is less than 2 moles, it becomes difficult to sufficiently lower the temperature range in which the liquid-crystallinity of the liquid-crystal urethane compound is exhibited, and there is a tendency that it is difficult to continuously form a thermally responsive liquid-crystal polyurethane elastomer while reaction-curing the urethane compound in a state in which liquid-crystallinity is exhibited without any solvent. On the other hand, when the number of moles added exceeds 10 moles, the liquid-crystal urethane compound tends to fail to exhibit the liquid-crystallinity.

The thermally responsive liquid-crystal polyurethane elastomer of the present invention is obtained by reacting a starting material composition for a thermally responsive liquid-crystal polyurethane elastomer which contains the liquid-crystal urethane compound and a polyfunctional compound having a functionality of 3 or more which reacts with an active hydrogen group or an isocyanate group of the liquid-crystal urethane compound. Said thermally responsive liquid-crystal polyurethane elastomer has liquid-crystallinity and rubber elasticity at low temperatures (in the vicinity of room temperature).

In the thermally responsive liquid-crystal polyurethane elastomer, the melting point of a mesogen does not exist between the glass transition temperature (Tg) and the transition temperature (Ti) from a liquid-crystal phase to an isotropic phase, and a liquid-crystal appears at a temperature of between Tg and Ti. The Ti is preferably from 0 to 100 °C.

In addition, the present invention relates to a method for producing a thermally responsive liquid-crystal polyurethane elastomer, comprising the step of stretching the starting material composition for a thermally responsive liquid-crystal polyurethane elastomer according to claim 5 while extruding the composition at a temperature at which liquid-crystallinity is exhibited and curing the composition in a state of being oriented the mesogenic group of the liquid-crystal urethane compound in the stretching direction.

## EFFECT OF THE INVENTION

The liquid-crystal urethane compound of the present invention has a low temperature range in which liquid-crystallinity is exhibited. By using the liquid-crystal urethane compound, it is possible to continuously form a thermally responsive liquid-crystal polyurethane elastomer while reaction-curing the liquid-crystal urethane compound in a state in which liquid-crystallinity is exhibited without any solvent. The thermally responsive liquid-crystal polyurethane elastomer of the present invention has a low temperature range in which the liquid-crystallinity of the liquid-crystal urethane compound as a starting material is exhibited and has a network structure by crosslinking, so that said elastomer has liquid-crystallinity and rubber elasticity at low temperatures (in the vicinity of room temperature) . Since the mesogenic group of the thermally responsive liquid-crystal polyurethane elastomer is oriented in a uniaxial direction, said polyurethane elastomer shows a characteristic response behavior such that it shrinks by a decrease in the degree of the mesogenic group orientation due to heat applied thereto and extends in the orientation direction by an increase in the degree of the mesogenic group orientation due to removal of the heat applied.

MODE FOR CARRYING OUT THE INVENTION

The liquid-crystal urethane compound of the present invention is one obtained by reacting a mesogenic group-containing compound having at least an active hydrogen group, an alkylene oxide and/or a styrene oxide, and a diisocyanate compound.

The mesogenic group-containing compound having an active hydrogen group is not particularly limited as long as it is a compound having an active hydrogen group and a mesogenic group, but said mesogenic group-containing compound is preferably a compound represented by the following general formula (1):

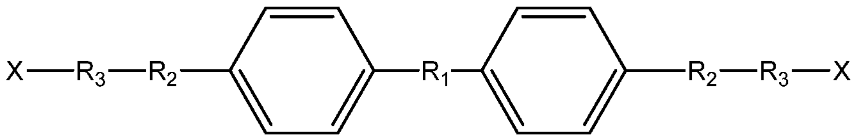

(1)

wherein X is an active hydrogen group, $R_1$ is a single bond, -N=N-, -CO-, -CO-O-, or -CH=N-, $R_2$ is a single bond or -O-, and $R_3$ is a single bond or an alkylene group having 1 to 20 carbon atoms, provided that the compound when $R_2$ is -O- and $R_3$ is a single bond is excluded.

Examples of X include OH, SH, $NH_2$, COOH, secondary amines, and the like.

In order to obtain a thermally responsive liquid-crystal polyurethane elastomer having a transition temperature (Ti) of 0 to 100 °C from a liquid-crystal phase to an isotropic phase or from the isotropic phase to the liquid-crystal phase, it is preferable to use a compound having a biphenyl skeleton ($R_1$ is a single bond) . When $R_3$ is an alkylene group, the number of carbon atoms is preferably 2 to 10.

The alkylene oxide to be added is not particularly limited, and examples thereof include ethylene oxide, propylene oxide, 1,2-butylene oxide, 2,3-butylene oxide, cyclohexene oxide, epichlorohydrin, epibromohydrin, methyl glycidyl ether, allyl glycidyl ether, and the like. The styrene oxide to be added may have a substituent such as an alkyl group, an alkoxyl group, or a halogen on the benzene ring.

In order to obtain a thermally responsive liquid-crystal polyurethane elastomer having a transition temperature (Ti) of 0 to 100 °C at which phase transition from a liquid-crystal phase to an isotropic phase or from the isotropic phase to the liquid-crystal phase occurs, it is preferable to use at least one oxide selected from the group consisting of ethylene oxide, propylene oxide, 1,2-butylene oxide, 2,3-butylene oxide, and styrene oxide.

The alkylene oxide and/or the styrene oxide is preferably added in an amount of 2 to 10 moles, more preferably 2 to 8 moles, with respect to 1 mole of the compound represented by the general formula (1).

The known compounds in the field of polyurethanes can be used as the diisocyanate compound without any particular limitation. The diisocyanate compounds include, for example, aromatic diisocyanates such as 2,4-toluene diisocyanate, 2,6-toluene diisocyanate, 2,2'-diphenyl methane diisocyanate, 2,4'-diphenyl methane diisocyanate, 4,4'-diphenyl methane diisocyanate, 1,5-naphthalene diisocyanate, p-phenylene diisocyanate, m-phenylene diisocyanate, p-xylylene diisocyanate and m-xylylene diisocyanate, aliphatic diisocyanates such as ethylene diisocyanate, 2,2,4-trimethyl hexamethylene-1,6-diisocyanate, 2,4,4-trimethyl hexamethylene-1,6-diisocyanate and 1,6-hexamethylene diisocyanate, and cycloaliphatic diisocyanates such as 1,4-cyclohexane diisocyanate, 4,4'-dicyclohexyl methane diisocyanate, isophorone diisocyanate and norbornane diisocyanate. These may be used alone or as a mixture of two or more thereof.

The blending ratio of the diisocyanate compound is preferably 10 to 40 wt%, more preferably 15 to 30 wt%, with respect to all the starting materials of the liquid-crystal urethane compound. When the blending ratio of the diisocyanate compound is less than 10 wt%, it tends to be difficult to continuously form the thermally responsive liquid-crystal polyurethane elastomer because of insufficient increase of the molecular weight in the urethanization reaction. On the other hand, when the blending ratio of the diisocyanate compound exceeds 40 wt%, the blending ratio of the mesogenic group-containing compound decreases, so that the liquid-crystal urethane compound tends to be difficult to exhibit the liquid-crystallinity.

In order to adjust the molecular weight or viscosity of the liquid-crystal urethane compound, an isocyanate compound having a functionality of 3 or more may be blended. The isocyanate compound having a functionality of 3 or more is not particularly limited, and examples thereof include compounds described later.

The liquid-crystal urethane compound may be produced by reacting a starting material composition containing at least the mesogenic group-containing compound, the alkylene oxide and/or the styrene oxide, and the diisocyanate compound. Alternatively, the liquid-crystal urethane compound may be produced by reacting the mesogenic group-containing compound with the alkylene oxide and/or the styrene oxide to obtain an oxide-added mesogenic group-containing compound, and reacting the diisocyanate compound or the like with the oxide-added mesogenic group-containing compound. The temperature at which the mesogenic group-containing compound is reacted with the alkylene

oxide and/or the styrene oxide is preferably about 110 to 130 °C. If the reaction temperature is less than 110 °C, the reaction tends to be difficult to proceed. If the reaction temperature exceeds 130 °C, side reactions tend to occur, and it tends to become difficult to obtain the desired oxide-added mesogenic group-containing compounds having hydroxyl groups at both ends.

The transition temperature (Ti) from a liquid-crystal phase to an isotropic phase or from the isotropic phase to the liquid-crystal phase of the liquid-crystal urethane compound is preferably from 15 to 150 °C, more preferably from 25 to 125 °C.

The thermally responsive liquid-crystal polyurethane elastomer of the present invention is one obtained by reacting a starting material composition for a thermally responsive liquid-crystal polyurethane elastomer, said composition containing the liquid-crystal urethane compound and a polyfunctional compound having a functionality of 3 or more which reacts with the active hydrogen group or the isocyanate group of the liquid-crystal urethane compound. The liquid-crystal urethane compound may be used singly or in combination of two or more kinds thereof.

The polyfunctional compound is a starting material for introducing a crosslinking point into the thermally responsive liquid-crystal polyurethane elastomer to form a network structure and imparting rubber elasticity to the thermally responsive liquid-crystal polyurethane elastomer.

The polyfunctional compound is not particularly limited as long as it is a compound having 3 or more functional groups reactive with the active hydrogen group or the isocyanate group of the liquid-crystal urethane compound, and examples thereof include an isocyanate compound having a functionality of 3 or more, an active hydrogen group-containing compound having a functionality of 3 or more, and the like.

The isocyanate compounds having a functionality of 3 or more include, for example, triisocyanates (e.g., triphenylmethane triisocyanate, tris(isocyanatephenyl)thiophosphate, lysine ester triisocyanate, 1,3,6-hexamethylene triisocyanate, 1,6,11-undecane triisocyanate, 1,8-diisocyanate-4-isocyanate methyloctane, and bicycloheptane triisocyanate) and tetraisocyanates (e.g., tetraisocyanate silane). These may be used alone or in combination of two or more thereof. It may also be possible to use a polymerized diisocyanate. As used herein, the term 'polymerized diisocyanate' refers to any of polymerized isocyanate derivatives produced by addition of three or more molecules of diisocyanate, or refers to a mixture of the isocyanate derivatives. For example, the isocyanate derivative may be of (1) trimethylolpropane adduct type, (2) biuret type, (3) isocyanurate type, or the like.

Examples of the active hydrogen group-containing compound having a functionality of 3 or more include high molecular weight polyols (molecular weight of 400 or more) having 3 or more hydroxyl groups, such as polyether polyol, polyester polyol, polycarbonate polyol, and polyester polycarbonate polyol; low molecular weight polyols such as trimethylolpropane, glycerin, 1,2,6-hexanetriol, pentaerythritol, tetramethylolcyclohexane, methylglucoside, sorbitol, mannitol, dulcitol, sucrose, 2,2,6,6-tetrakis(hydroxymethyl)cyclohexanol, and triethanolamine; low molecular weight polyamines such as diethylenetriamine; and the like. These may be used singly or in combination of two or more kinds thereof.

The blending ratio of the polyfunctional compound is preferably 2 to 20 wt%, more preferably 4 to 10 wt%, with respect to all the starting materials of the thermally responsive liquid-crystal polyurethane elastomer. When the blending ratio of the polyfunctional compound is less than 2 wt%, memory of the orientation state after orientation of the mesogenic groups is lowered, so that reversible shape deformation (thermal responsiveness) tends to be lost. On the other hand, when the blending ratio of the polyfunctional compound exceeds 20 wt%, the crosslinking density becomes higher, so that the glass transition temperature rises and the temperature range in which the liquid-crystallinity is exhibited becomes narrower. Therefore, it tends to be difficult to continuously form a liquid-crystal polyurethane elastomer having thermal responsibility. In addition, the polyurethane elastomer tends to be difficult to exhibit the liquid-crystallinity.

The thermally responsive liquid-crystal polyurethane elastomer may be produced by a batch method or may be produced by a continuous production method, but from the viewpoint of industrial production efficiency, it is preferable to produce such a polyurethane elastomer by a continuous production method.

In the case of a batch method, the liquid-crystal urethane compound is crosslinked with the polyfunctional compound and cured to produce a thermally responsive liquid-crystal polyurethane elastomer. During the crosslinking reaction, the mesogenic group of the liquid-crystal urethane compound is oriented in a uniaxial direction while the liquid-crystal urethane compound exhibits the liquid-crystallinity, and is cured in a state of being oriented. There is no particular limitation on the method of orienting the mesogenic group in the uniaxial direction, and examples of such a method include a method of performing a crosslinking reaction on the oriented film, a method of orientation by applying an electric or magnetic field at the time of crosslinking reaction, a method of stretching in the semi-cured state.

In the case of the continuous production method, the starting material composition is stretched while extruding at a temperature at which liquid-crystallinity is exhibited, and the mesogenic group of the liquid-crystal urethane compound is cured in a state of being oriented in the stretching direction, whereby a thermally responsive liquid-crystal polyurethane elastomer is obtained. The viscosity of the liquid-crystal urethane compound in a temperature range where liquid-crystallinity is exhibited is preferably 10 to 5000 Pa•s, more preferably 100 to 2000 Pa•s. When the viscosity is less than 10 Pa•s, the oriented state of the mesogenic group at the time of stretching tends to decrease due to relaxation, and

when the viscosity exceeds 5000 Pa•s, stretching becomes difficult, so that the mesogen group tends to be difficult to be highly oriented. Further, the molding temperature is preferably in the vicinity of the transition temperature (Ti) of the liquid-crystal urethane compound. In addition, the stretch ratio is preferably about 150 to 500%. When the stretch ratio is less than 150%, it tends to be difficult to obtain a liquid-crystal polyurethane elastomer deformed by a thermal response. On the other hand, when the stretch ratio exceeds 500%, there is a high possibility that the thermally responsive liquid-crystal polyurethane elastomer will rupture during molding, because of which continuous production tends to be difficult.

In the thermally responsive liquid-crystal polyurethane elastomer of the present invention, the melting point of a mesogen does not exist between the glass transition temperature (Tg) and the transition temperature (Ti) from a liquid-crystal phase to an isotropic phase, and a liquid-crystal appears at a temperature of between Tg and Ti. The transition temperature (Ti) is preferably from 0 to 100 °C, more preferably from 0 to 85 °C.

## EXAMPLES

Description will be given of the invention with examples, while the invention is not limited to description in the examples.

### Measurement and Evaluation Method

(Measurement of Glass Transition Temperature (Tg) of Liquid-crystal Urethane Compound and Thermally Responsive Liquid-crystal Polyurethane Elastomer, Melting Point (Tm) of Mesogen, and (Liquid-Crystal Phase)-to-(Isotropic Phase) Transition Temperature (Ti))

The Tg, Tm, and Ti were measured under the condition of 20 °C/min using a differential scanning calorimeter DSC (manufactured by Hitachi High-Tech Science Corp., trade name: X-DSC 7000).

### Measurement of Viscosity of Liquid-crystal Urethane Compound

The viscosity of the liquid-crystal urethane compound was measured using a capillary rheometer (trade name: SemiAutomatic Capillary Rheometer (SAS-2002), manufactured by Yasuda Seiki Seisakusho, Ltd.) in accordance with JIS K 7199 at 60 °C and a shear rate of 1000 $sec^{-1}$.

### Evaluation of Liquid Crystallinity

The presence or absence of liquid crystallinity of the liquid-crystal urethane compound and thermally responsive liquid-crystal polyurethane elastomer were evaluated by using a polarization microscope (manufactured by Nikon Corporation, trade name: LV-100POL) and a differential scanning calorimeter DSC (manufactured by Hitachi High-Tech Science Corp., trade name: X-DSC 7000) under the condition of 20 °C/min.

### Measurement of Degree of Orientation of Thermally Responsive Liquid-crystal Polyurethane Elastomer

The degree of orientation of the thermally responsive liquid-crystal polyurethane elastomer was calculated by the following formula. Absorbance was measured under the conditions of single reflection ATR, germanium IRE, the incident angle of 45°, and vertical polarization using an FT-IR (trade name: Spectrum 100, manufactured by Perkin Elmer, Inc.). The arrangement of the sample was arbitrary two orthogonal directions (0°, 90°). A large value of degree of orientation means that the mesogenic groups are highly oriented in the uniaxial direction. The degree of orientation is preferably 0.1 or more, more preferably 0.2 or more.

$$\text{Degree of orientation of thermally responsive liquid-crystal polyurethane elastomer} = (A - B)/(A + 2B)$$

A = (Absorbance of antisymmetric stretching vibration of aromatic ether as measured at 0°)/(Absorbance of symmetric deformation vibration of methyl group as measured at 0°)

B = (Absorbance of antisymmetric stretching vibration of aromatic ether as measured at 90°)/(Absorbance of symmetric deformation vibration of methyl group as measured at 90°)

Example 1

BH4 (500 g), KOH 19.0 g, and DMF 3000 ml were placed in a reaction vessel and mixed, then 2 equivalents of propylene oxide were added to BH4 (1 mole) . The mixture was reacted under pressurized conditions at 120 °C for 2 hours. Thereafter, oxalic acid 15.0 g was added thereto to stop the addition reaction, and the salt was removed by suction filtration, after which DMF was further removed by distillation under reduced pressure to obtain the desired mesogenic diol A (which may contain a structural isomer). The reaction is shown below.

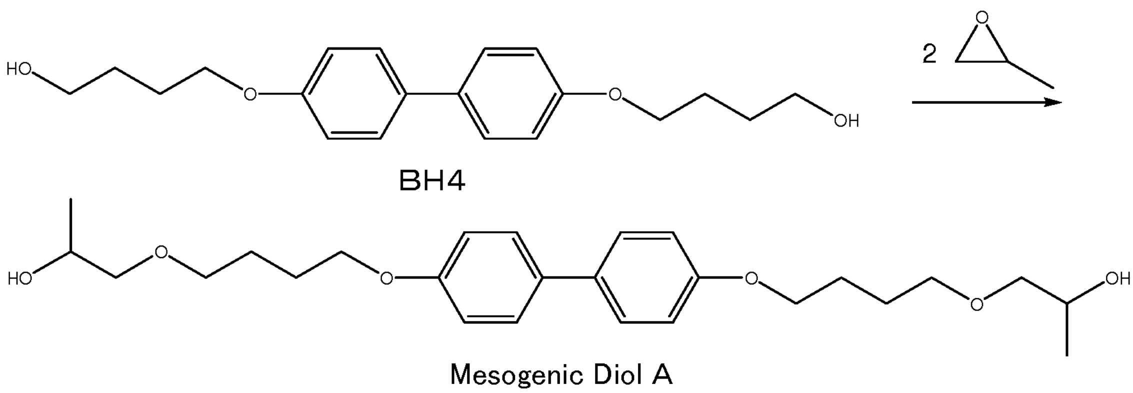


BH4

Mesogenic Diol A

The mesogenic diol A 500 g, hexamethylene diisocyanate (HDI) 154 g, and a catalyst (TEDA-L33, manufactured by Tosoh Corporation) 5 g were mixed, and the mixture was allowed to react at 100 °C for 2 hours to obtain a liquid-crystal urethane compound A1 (NCO index: 0.9).

While adding 4.9 parts by weight of a trifunctional isocyanurate (SUMIDUR N3300, manufactured by Sumika Bayer Urethane Co., Ltd.) to the liquid-crystal urethane compound A1 (100 parts by weight) with a side feeder, the mixture was extruded with a thickness of 1 mm × width of 100 mm at 60 °C, then molded while stretching to 200% to prepare a thermally responsive liquid-crystal polyurethane elastomer.

Example 2

Mesogenic diol B (which may include a structural isomer) was obtained in the same manner as in Example 1, except that 3 equivalents of propylene oxide were added to BH4 (1 mole). The mesogenic diol B 500 g, hexamethylene diisocyanate (HDI) 137 g, and a catalyst (TEDA-L33, manufactured by Tosoh Corporation) 5 g were mixed and the mixture was reacted at 100 °C for 2 hours to obtain a liquid-crystal urethane compound B1 (NCO index: 0.9). While adding 4.3 parts by weight of a trifunctional isocyanurate (SUMIDUR N3300, manufactured by Sumika Bayer Urethane Co., Ltd.) to the liquid-crystal urethane compound B1 (100 parts by weight) with a side feeder, the mixture was extruded with a thickness of 1 mm × width of 100 mm at 60 °C, then molded while stretching to 200% to prepare a thermally responsive liquid-crystal polyurethane elastomer.

Example 3

BH6 (500 g), KOH 19.0 g, and DMF 3000 ml were placed in a reaction vessel and mixed, then 2 equivalents of propylene oxide were added to BH6 (1 mole) . The mixture was reacted under pressurized conditions at 120 °C for 2 hours. Thereafter, oxalic acid 15.0 g was added thereto to stop the addition reaction, and the salt was removed by suction filtration, after which DMF was further removed by distillation under reduced pressure to obtain the desired mesogenic diol C (which may contain a structural isomer). The reaction is shown below.

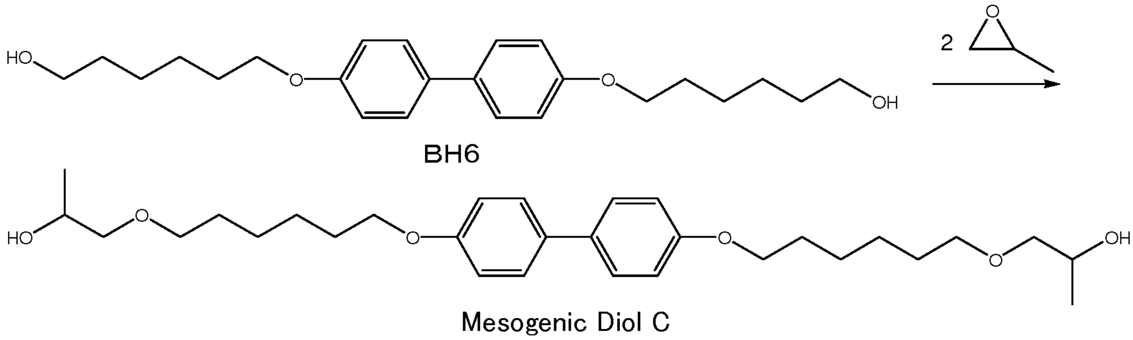


BH6

Mesogenic Diol C

The mesogenic diol C 500 g, a mixture of 2,2,4-trimethylhexamethylene-1,6-diisocyanate and 2,4,4-trimethylhexamethylene-1,6-diisocyanate (VESTANAT TMDI, manufactured by Evonik Degussa) 188 g, and a catalyst (TEDA-L33, manufactured by Tosoh Corporation) 5 g were mixed and the mixture was reacted at 100 °C for 2 hours to obtain a liquid-crystal urethane compound C1 (NCO index: 0.9).

While adding 4.3 parts by weight of a trifunctional isocyanurate (SUMIDUR N3300, manufactured by Sumika Bayer Urethane Co., Ltd.) to the liquid-crystal urethane compound C1 (100 parts by weight) with a side feeder, the mixture was extruded with a thickness of 1 mm × width of 100 mm at 60 °C, then molded while stretching to 200% to prepare a thermally responsive liquid-crystal polyurethane elastomer.

Example 4

The mesogenic diol C 500 g, VESTANART TMDI (168 g) and a catalyst (TEDA-L33, manufactured by Tosoh Corporation) 5 g were mixed and the mixture was reacted at 100 °C for 2 hours to obtain a liquid-crystal urethane compound C2 (NCO index: 0.8).

While adding 8.9 parts by weight of a trifunctional isocyanurate (SUMIDUR N3300, manufactured by Sumika Bayer Urethane Co., Ltd.) to the liquid-crystal urethane compound C2 (100 parts by weight) with a side feeder, the mixture was extruded with a thickness of 1 mm × width of 100 mm at 60 °C, then molded while stretching to 200% to prepare a thermally responsive liquid-crystal polyurethane elastomer.

Example 5

The mesogenic diol C 500 g, VESTANART TMDI (199 g) and a catalyst (TEDA-L33, manufactured by Tosoh Corporation) 5 g were mixed and the mixture was reacted at 100 °C for 2 hours to obtain a liquid-crystal urethane compound C3 (NCO index: 0.95) .

While adding 2.2 parts by weight of a trifunctional isocyanurate (SUMIDUR N3300, manufactured by Sumika Bayer Urethane Co., Ltd.) to the liquid-crystal urethane compound C3 (100 parts by weight) with a side feeder, the mixture was extruded with a thickness of 1 mm × width of 100 mm at 60 °C, then molded while stretching to 200% to prepare a thermally responsive liquid-crystal polyurethane elastomer.

Table 1

| | Characteristics of liquid-crystal urethane compound | | | | | Characteristics of thermally responsive liquid-crystal polyurethane elastomer | | | | | |
|---|---|---|---|---|---|---|---|---|---|---|---|
| | Glass transition temperature (Tg) | Melting point (Tm) | Transition temperature (Ti) | Liquid-crystallinity | Viscosity at 60°C (Pa·s) | Glass transition temperature (Tg) | Melting point (Tm) | Transition temperature (Ti) | Liquid-crystallinity | Degree of orientation | Reversible deformation by thermal response |
| Example 1 | -7 | - | 77 | Yes | 450 | 10 | - | 65 | Yes | 0.21 | Yes |
| Example 2 | -8 | - | 69 | Yes | 350 | 11 | - | 55 | Yes | 0.18 | Yes |
| Example 3 | -9 | - | 62 | Yes | 280 | 15 | - | 40 | Yes | 0.14 | Yes |
| Example 4 | -8 | - | 65 | Yes | 40 | 18 | - | 38 | Yes | 0.11 | Yes |
| Example 5 | -9 | - | 61 | Yes | 1120 | 11 | - | 47 | Yes | 0.24 | Yes |

INDUSTRIAL APPLICABILITY

Since the thermally responsive liquid-crystal polyurethane elastomer of the present invention has a characteristic response behavior such that said elastomer shrinks by a decrease in the degree of the liquid-crystal orientation due to heat applied thereto and extends in the orientation direction by an increase in the degree of the liquid-crystal orientation due to removal of the heat applied, it is possible to apply the liquid-crystal polyurethane elastomer to various fields such as actuators.

## Claims

1. A liquid-crystal urethane compound obtained by reacting a mesogenic group-containing compound having at least an active hydrogen group, an alkylene oxide and/or a styrene oxide, and a diisocyanate compound.

2. The liquid-crystal urethane compound according to claim 1, wherein the mesogenic group-containing compound is a compound represented by the following general formula (1):

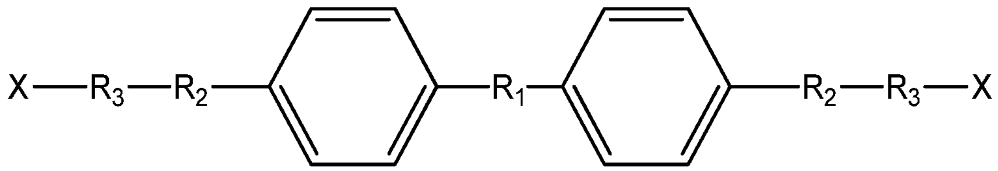

(1)

wherein X is an active hydrogen group, $R_1$ is a single bond, -N=N-, -CO-, -CO-O-, or -CH=N-, $R_2$ is a single bond or -O-, and $R_3$ is a single bond or an alkylene group having 1 to 20 carbon atoms, provided that the compound when $R_2$ is -O- and $R_3$ is a single bond is excluded.

3. The liquid-crystal urethane compound according to claim 1 or 2, wherein the alkylene oxide is at least one member selected from the group consisting of ethylene oxide, propylene oxide, and butylene oxide.

4. The liquid-crystal urethane compound according to any one of claims 1 to 3, wherein 2 to 10 moles of the alkylene oxide and/or the styrene oxide are added to 1 mole of the mesogenic group-containing compound.

5. A starting material composition for a thermally responsive liquid-crystal polyurethane elastomer, which contains the liquid-crystal urethane compound according to any one of claims 1 to 4 and a polyfunctional compound having a functionality of 3 or more which reacts with an active hydrogen group or an isocyanate group of the liquid-crystal urethane compound.

6. A thermally responsive liquid-crystal polyurethane elastomer obtained by reacting the starting material composition for a thermally responsive liquid-crystal polyurethane elastomer according to claim 5.

7. The thermally responsive liquid-crystal polyurethane elastomer according to claim 6, wherein the melting point of a mesogen does not exist between the glass transition temperature (Tg) and the transition temperature (Ti) from a liquid-crystal phase to an isotropic phase, and a liquid-crystal appears at a temperature of between Tg and Ti.

8. The thermally responsive liquid-crystal polyurethane elastomer according to claim 7, wherein the Ti is from 0 to 100 °C.

9. A method for producing a thermally responsive liquid-crystal polyurethane elastomer, comprising the step of stretching the starting material composition for a thermally responsive liquid-crystal polyurethane elastomer according to claim 5 while extruding the composition at a temperature at which liquid-crystallinity is exhibited and curing the composition in a state of being oriented the mesogenic group of the liquid-crystal urethane compound in the stretching direction.

**INTERNATIONAL SEARCH REPORT**

International application No.
PCT/JP2016/068534

A. CLASSIFICATION OF SUBJECT MATTER
*C08G18/32*(2006.01)i

According to International Patent Classification (IPC) or to both national classification and IPC

B. FIELDS SEARCHED

Minimum documentation searched (classification system followed by classification symbols)
C08G18/00-18/87, C09K19/00-19/60, C08J3/00-7/18

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 7-258369 A (Nitta Corp.),<br>09 October 1995 (09.10.1995),<br>claims; examples<br>(Family: none) | 1-4<br>5-9 |
| X<br>Y | JP 4-332716 A (Mitsui Toatsu Chemicals, Inc.),<br>19 November 1992 (19.11.1992),<br>claims; examples<br>(Family: none) | 1-4<br>5-9 |
| X<br>Y | JP 63-51413 A (Bayer AG.),<br>04 March 1988 (04.03.1988),<br>claims; examples<br>& US 4791187 A<br>claims; examples<br>& EP 256470 A2 | 1-4<br>5-9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 September 2016 (12.09.16) | 27 September 2016 (27.09.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

INTERNATIONAL SEARCH REPORT

International application No. PCT/JP2016/068534

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2014-172967 A (Toyo Tire and Rubber Co., Ltd.), 22 September 2014 (22.09.2014), claims; examples (Family: none) | 5-9 |
| Y | WO 2015/093099 A1 (Toyo Tire and Rubber Co., Ltd.), 25 June 2015 (25.06.2015), claims; examples & JP 2015-117296 A & JP 2015-117295 A | 5-9 |
| P,X<br>P,A | JP 2016-58162 A (Toyo Tire and Rubber Co., Ltd.), 21 April 2016 (21.04.2016), preparation examples 1 to 2 (Family: none) | 6-8<br>1-5,9 |
| P,X<br>P,A | JP 2016-56352 A (Toyo Tire and Rubber Co., Ltd.), 21 April 2016 (21.04.2016), examples (Family: none) | 6-8<br>1-5,9 |
| P,X<br>P,A | JP 2016-56345 A (Toyo Tire and Rubber Co., Ltd.), 21 April 2016 (21.04.2016), examples (Family: none) | 6-8<br>1-5,9 |
| P,X<br>P,A | JP 2016-56113 A (Toyo Tire and Rubber Co., Ltd.), 21 April 2016 (21.04.2016), examples (Family: none) | 6-8<br>1-5,9 |
| A | JP 2005-194427 A (Fuji Photo Film Co., Ltd.), 21 July 2005 (21.07.2005), (Family: none) | 1-9 |
| A | JP 2013-245321 A (Oita University), 09 December 2013 (09.12.2013), (Family: none) | 1-9 |
| A | JP 11-198541 A (Asahi Chemical Industry Co., Ltd.), 27 July 1999 (27.07.1999), (Family: none) | 1-9 |
| A | JP 5-239194 A (Moriyuki SATO), 17 September 1993 (17.09.1993), (Family: none) | 1-9 |

Form PCT/ISA/210 (continuation of second sheet) (January 2015)

**INTERNATIONAL SEARCH REPORT**

International application No.
PCT/JP2016/068534

C (Continuation). DOCUMENTS CONSIDERED TO BE RELEVANT

| Category* | Citation of document, with indication, where appropriate, of the relevant passages | Relevant to claim No. |
|---|---|---|
| A | JP 7-316498 A (NKK Corp.), 05 December 1995 (05.12.1995), & US 5817731 A & US 6018013 A | 1-9 |

Form PCT/ISA/210 (continuation of second sheet) (January 2015)

**INTERNATIONAL SEARCH REPORT**

International application No.
PCT/JP2016/068534

Notice concerning the prior art search:

This prior art search has been carried out on the assumption that claim 6 refers to claim 5.

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H5170860 A
- JP H7258369 A